# EUROPÄISCHE PATENTSCHRIFT

(11) **EP 0 856 282 B1**
(45) Veröffentlichungstag und Bekanntmachung des Hinweises auf die Patenterteilung: **17.03.2004**
(21) Anmeldenummer: 97122101.5
(22) Anmeldetag: 15.12.1997
(51) Int. Cl.: A61B 6/08, G03B 42/02

(54) **Röntgenaufnahmevorrichtung**
Radiographic apparatus
Appareil radiographique

(30) Priorität: 30.01.1997 SE 9700277
(43) Veröffentlichungstag der Anmeldung: 05.08.1998
(73) Patentinhaber: Siemens Aktiengesellschaft, 80333 München (DE)
(72) Erfinder: Thunberg, Stefan, 115 35 Stockholm (SE)

(56) Entgegenhaltungen:
- US-A- 4 092 544
- PATENT ABSTRACTS OF JAPAN vol. 013, no. 111 (C-577), 16.März 1989 & JP 63 286170 A (NEC CORP), 22.November 1988,

## Beschreibung

Die Erfindung betrifft eine Röntgenaufnahmevorrichtung umfassend einen Strahlenkopf mit einer Röntgenröhre, die mit Kollimatoren zur Begrenzung eines ausgestrahlten Röntgenstrahlenbündels versehen ist, bei dem der Strahlenkopf zur Ausrichtung des Strahlenbündels im Raum translatorisch verschiebbar und/oder in Winkelrichtung drehbar ist, sowie eine digitale Bildplatte, bestehend aus einer Mehrzahl von Pixeln, wobei eine mechanische Verbindung zwischen der Bildplatte und dem Strahlenkopf fehlt.

Bei Röntgenuntersuchungen von Patienten ist es wichtig, daß das Strahlenbündel, das von der Strahlenquelle ausgestrahlt wird, nach dem Passieren desjenigen Teils des Patienten, der geröngt werden soll, den Röntgenfilm trifft, damit ein Bild der aufgenommenen Stelle erzeugt wird. Bei z.B. mobilen Röntgengeräten, z.B. des Typs, der in dem Prospekt MOBILETT Plus der Fa. Siemens-Elema AB beschrieben worden ist, wird eine Filmkassette ohne eine mechanische Verbindung mit der Röntgenstrahlenquelle unterhalb des Patienten, häufig unterhalb der Matratze, auf der der Patient gelagert ist, plaziert. Der Operateur richtet danach das Strahlenfeld nach Gefühl gegen die Filmkassette, wobei das Strahlenfeld häufig extra groß gemacht wird, um nicht zu riskieren, außerhalb des Films zu liegen. Dies führt zu unnötig hohen Strahlendosen und aufgrund von Streustrahlung zu schlechterer Bildqualität. Dies führt außerdem aufgrund falscher Einstellungen des Strahlenfeldes im Verhältnis zur Kassette zu Aufnahmewiederholungen.

Ein Versuch, mit diesem Problem zurecht zu kommen, ist in der US-A, 4 092 544 beschrieben. In dieser Schrift ist eine Röntgenaufnahmevorrichtung mit einer Lichtquelle beschrieben, die im Verhältnis zur Röntgenröhre und deren Blende fest verbunden ist. Es sind weiterhin an einem mit der Filmkassette fest verbundenen Teil Markierungen angeordnet. Die Lichtquelle und das erwähnte mit Markierungen versehene Teil der Filmkassette sind so angeordnet, daß das Licht der Lichtquelle von einem Patient, der mit der Röntgenvorrichtung geröntgt wird, nicht unterbrochen wird. Durch Beobachten, wo das Lichtbündel von der Lichtquelle das mit der Filmkassette fest verbundene Teil trifft sowie wie das Lichtbündel die Markierungen trifft, kann festgestellt werden, ob die Kanten der Filmkassette mit den äußeren Begrenzungen desjenigen Röntgenstrahlenbündels, das die Filmkassette trifft, parallel sind, ob der Zentralstrahl des Röntgenstrahlenbündels die Mitte der Filmkassette trifft und ob ein gewünschter Fokusfilmabstand eingestellt worden ist.

In der US, A, 4 167 675 ist ein Röntgenkollimator beschrieben, bei dem mit Hilfe einer Lichtquelle ein Strahlenbündel erzeugt wird, dessen Form und Lage mit der Form und Lage des Röntgenstrahlenbündels übereinstimmt. Hierdurch wird eine visuelle Anzeige der Erstreckung und Lage des Röntgenstrahlenbündels erhalten. Diese visuelle Anzeige kann jedoch nicht zum Einstellen des Röntgenstrahlenbündels auf einer Filmkassette in dem Fall verwendet werden, indem der Bereich des Patienten, der aufgenommen werden soll, sich eventuell mit einer Matratze zwischen der Röntgenstrahlenquelle und der Filmkassette befindet.

Der Erfindung liegt die Aufgabe zugrunde, die oben erwähnten Probleme zu beseitigen, nämlich das Röntgenstrahlenbündel bei digitaler Bildregistrierung auf die Bildoberfläche zu richten und zu justieren.

Diese Aufgabe wird mit Hilfe einer Röntgenaufnahmevorrichtung der eingangs genannten Art mit den in dem Patentanspruch 1 genannten Merkmalen erreicht.

Bei einer Röntgenaufnahmevorrichtung nach der Erfindung wird also die Lage des Röntgenstrahlenbündels auf der digitalen Bildplatte bestimmt, indem die Signale der Bildplatte abgetastet werden, um zu bestimmen, welche Pixel bei einer Probeaufnahme von dem Strahlenbündel beleuchtet sind. Diese Information wird dann zum Einstellen der Kollimatoren der Röntgenstrahlenquelle bzw. zum Einstellen der Richtung und Lage des Strahlenkopfes im Verhältnis zur Bildplatte für eine optimale Einstellung des Strahlenbündels auf der Bildplatte benutzt.

Nach vorteilhaften Ausführungsformen der Vorrichtung nach der Erfindung kann die Einstellung des Röntgenstrahlenbündels auf der Bildplatte automatisiert werden, indem die Kollimatoren motorgetrieben sind, die Einstellmittel so angeordnet sind, daß sie automatisch die Motoren der Kollimatoren in Abhängigkeit einer abgetasteten Beleuchtung der Bildplatte steuern, und die Verschieb- und Drehmittel weiterhin eine motorgetriebene Antriebseinheit zum automatischen Einstellen des Strahlenkopfes in Abhängigkeit einer abgetasteten Beleuchtung der Bildplatte steuern.

Nach einer weiteren vorteilhaften Ausführungsform der Anordnung nach der Erfindung ist die Abtasteinheit dazu vorgesehen, bei einer Probeaufnahme die erhaltenen äußeren Konturen eines Patienten zu bestimmen, und die Einstell- und Drehmittel sind dazu vorgesehen, das Strahlenfeld in Abhängigkeit von den Steuersignalen der Abtasteinheit auf der Bildplatte an die äußeren Konturen des Patienten anzupassen. Auf diese Weise kann das Röntgenstrahlenfeld begrenzt werden, so daß lediglich der interessante Teil der Bildplatte bedeckt wird, d.h. die benutzte Strahlendosis kann reduziert werden. Ferner wird durch Verminderung der Streustrahlung die Bildqualität verbessert.

Die Erfindung ist nachfolgend anhand eines in den Zeichnungen dargestellten Ausführungsbeispiels näher erläutert. Es zeigen:
- FIG 1: die Verwendung eines mobilen Röntgengerätes,
- FIG 2: ein mobiles Röntgengerät, bei dem die Möglichkeit gezeigt wird, den Strahlenkopf zu bewegen,
- FIG 3: schematisch eine Röntgenstrahlenquelle, die eine digitale Bildplatte beleuchtet,
- FIG 4 und 5: schematisch eine Verschiebung des Strahlenkopfes, parallel mit der Bildplatte in zwei orthogonalen Richtungen,
- FIG 6: in einem Blockschaltbild die Steuerung der Röntgenstrahlenquelle und
- FIG 7 bis 9: Beispiele der Anpassung des Röntgenstrahlenbündels an den aktiven Teil der Bildplatte.

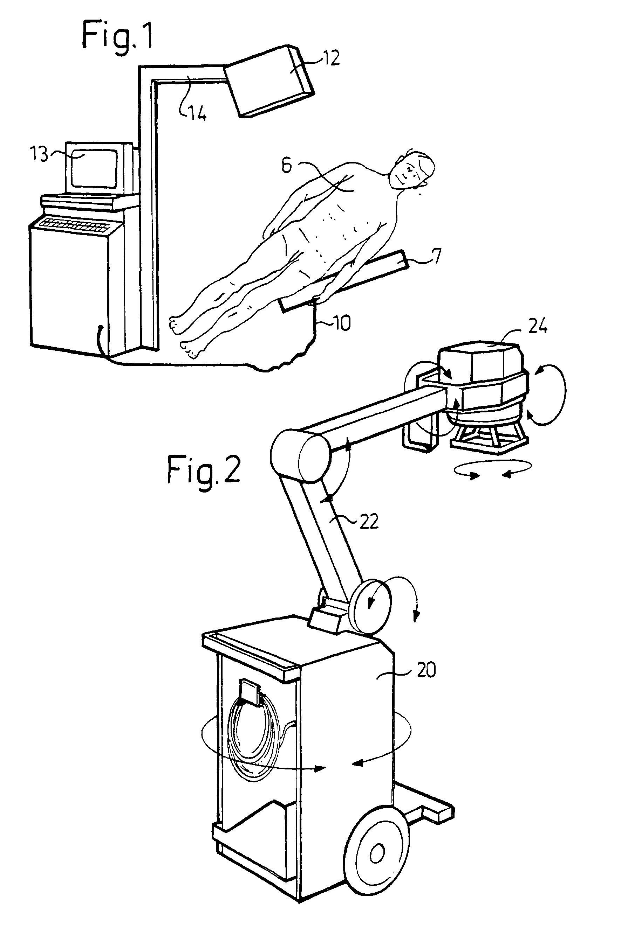

In der FIG 1 ist ein Röntgengerät mit einem beweglichen Strahlenkopf 12, der von einem Arm 14 getragen wird, gezeigt. Der Strahlenkopf 12 umfaßt eine Röntgenstrahlenquelle, mit der eine Röntgenaufnahme eines Patienten 6 vorgenommen werden kann. Unterhalb des Patienten 6 ist eine digitale Bildplatte 7 zum Registrieren des Röntgenbildes angeordnet. Die Bildplatte 7 besteht aus einer großen Anzahl von Pixeln oder Bildelementen in der Größenordnung 100 bis 200 µm und die Bildplatte 7 ist über ein Kabel 10 mit der Elektronik des Röntgengerätes zur Bearbeitung von Bilddaten verbunden. Das Röntgengerät ist auch mit einem PC 13 ausgerüstet. Die Verwendung des PCs 13 wird unten näher beschrieben.

In der FIG 2 ist ein mobiles Röntgengerät 20 gezeigt. Die Vorrichtung nach der Erfindung kann in Verbindung mit diesem Röntgengerät 20 verwendet werden. Das mobile Röntgengerät 20 ist normaler Weise dafür vorgesehen, in Verbindung mit einer Röntgenaufnahme an ein Bett eines Patienten gerollt zu werden. Die Möglichkeit, das Gerät 20 und dessen Arm 22, der den Strahlenkopf 24 trägt, sowie die Möglichkeit, den Strahlenkopf 24 selbst zum Einstellen des Röntgenstrahlenbündels zu bewegen, wird in der FIG mit Hilfe von Pfeilen gezeigt.

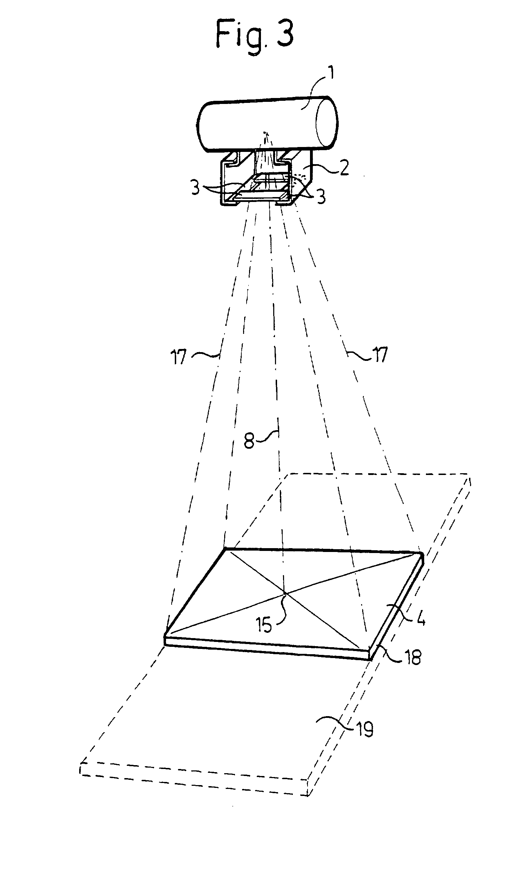

Der Strahlenkopf 24 beinhaltet eine Röntgenstrahlenquelle umfassend eine Röntgenröhre 1 mit einem Kollimator 2 in Form von untereinander senkrechten, beweglichen Blendenplatten 3, mit denen ein im wesentlichen rechteckförmiges Röntgenstrahlenbündel 17 abgeblendet werden kann. Dies ist aus der FIG 3 ersichtlich. Das Röntgenstrahlenbündel 17 trifft auf eine digitale Bildplatte 4, die auf einem Untersuchungstisch 19 angeordnet ist. In der FIG 3 ist gezeigt, daß das Strahlenbündel 17 auf der Bildplatte 4 zentriert und dessen Einfallswinkel senkrecht zur Bildplatte 4 gerichtet ist, d.h., daß der Zentralstrahl 8 senkrecht zur Bildplatte 4 in deren Mittelpunkt 15 auftrifft.

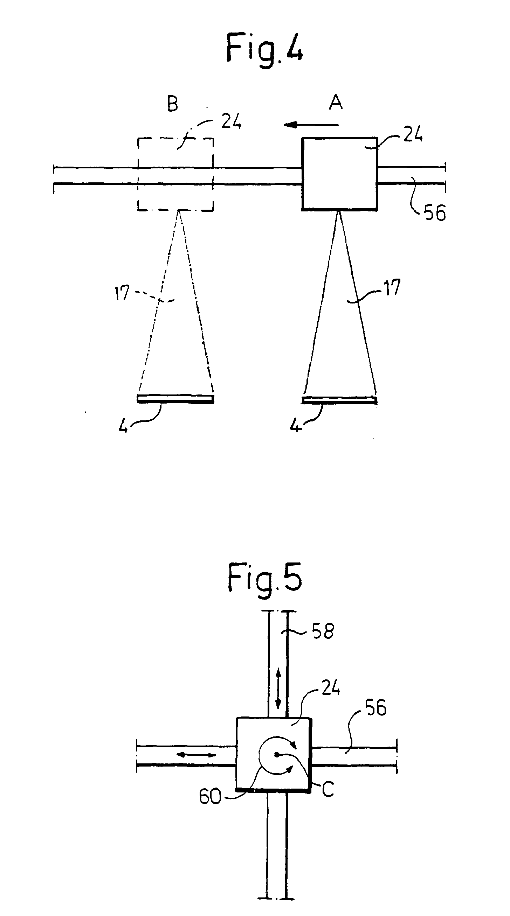

Zur Zentrierung des Strahlenbündels 17 auf der Bildplatte 4 kann der Strahlenkopf 24 auf Anordnungen 56, 58 montiert sein, die auch im Verhältnis zur Bildplatte 4 Parallelverschiebungen des Strahlenkopfes in zwei Dimensionen, wie es in den FIG 4 und 5 dargestellt ist, ermöglichen. In der FIG 4 wird demnach gezeigt, wie der Strahlenkopf 24 von der Position A zu der Position B versetzt wird, damit das Strahlenbündel auf der Bildplatte 4 zentriert wird.

Die Parallelverschiebung des Strahlenkopfes kann sowohl automatisch als auch manuell vorgenommen werden.

Die Anordnungen 56, 58, bei denen der Strahlenkopf 25 in einer Ebene und entlang zweier senkrechter Richtungen verlaufen kann, sind ihrerseits vorzugsweise im ganzen um ihren Kreuzpunkt C drehbar angeordnet. Dies ist in der FIG 5 mit dem Pfeil 60 gezeigt.

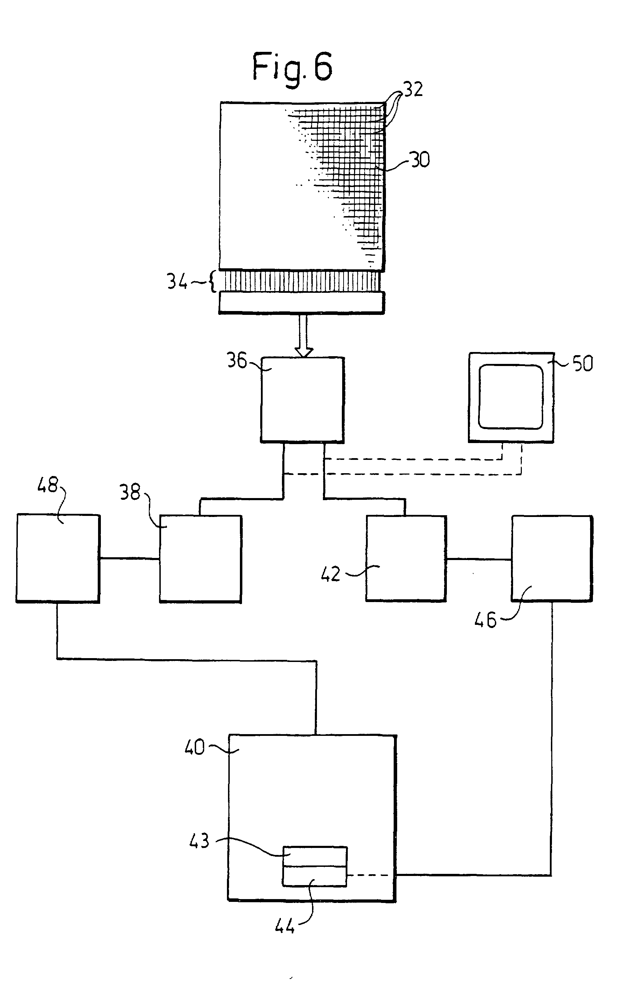

In der FIG 6 ist eine digitale Bildplatte 30 schematisch gezeigt, die aus Pixeln 32 aufgebaut ist. Jeder Pixel gibt bei Belichtung mit γ-Strahlung ein elektrisches Signal ab. Diese Signale sind über Anschlüsse 34 abnehmbar und werden einer Abtasteinheit 36, durch die abgetastet wird, welche Pixel bei einer Belichtung der Bildplatte 30 vom Röntgenstrahlenbündel bestrahlt werden, zugeführt. In Abhängigkeit von den Signalen der Pixel 32 der Bildplatte 30, gibt die Abtasteinheit 36 Steuersignale an Verschieb- und Drehmittel 38 zum Parallelverschieben und Ausrichten des Strahlenkopfes 40 im Raum sowie an Einstellmitteln 42 zum Einstellen der Kollimatoren 44 für die Röntgenstrahlenquelle 43 des Strahlenkopfes 40 ab. Bei einer Probebelichtung der Bildplatte 30 wird daher auf diese Weise eine optimale Einstellung des Röntgenstrahlenbündels auf der Bildplatte 30 mit Hilfe der bei der Probeaufnahme erzeugten Pixelsignale ermöglicht.

Die Kollimatoren 44 sind vorzugsweise motorgetrieben, wobei die Einstellmittel 42 den Kollimatormotor 46 abhängig von einer abgefühlten Belichtung der Pixel 32 der Bildplatte 30 steuern. Auch die Verschieb- und Drehmittel 38 steuern vorzugsweise eine motorgetriebene Antriebseinheit 48 zum automatischen Verschieben und Ausrichten des Strahlenkopfes 40 abhängig von einer abgefühlten Belichtung der Bildplatte 30.

Ein Bildschirm 50 kann an einer Abtasteinheit 36 angeschlossen sein, um auf dem Bildschirm 50 ein Bild des Strahlenfeldes auf der Bildplatte 30 zu zeigen. Bei dieser Ausführung können anhand des auf dem Bildschirm 50 gezeigten Bildes die Kollimatoren eingestellt und der Strahlenkopf manuell verschoben und/oder ausgerichtet werden, so daß eine optimale Einstellung des Röntgenstrahlenfeldes auf der Bildplatte erhalten wird. In einer praktischen Ausführung ist der Bildschirm 50 vorzugsweise ein PC, Bezugszeichen 13 in der FIG 1, dem die Bildinformation von der Bildplatte 7 über das Kabel 10 zugeführt wird.

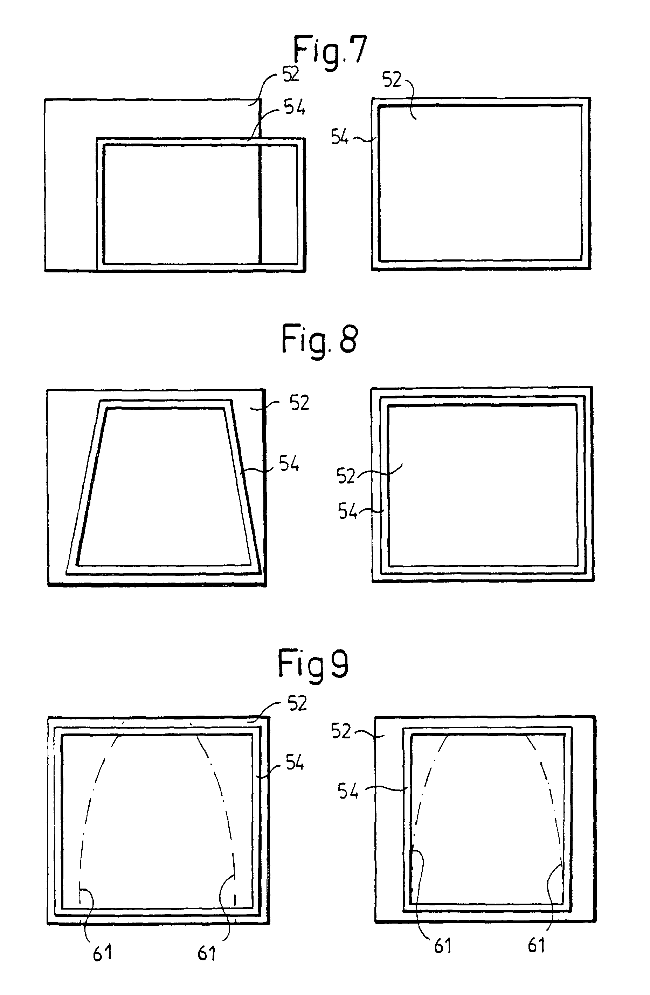

In den FIG. 7 und 8 werden Beispiele der Bildplatte mit Strahlenfedern gezeigt, wobei gewünscht wird, daß das Röntgenstrahlenbündel im wesentlichen die gesamte digitale Bildplatte 52 ausleuchten soll. Im linken Teil der FIG 7 und 8 wird durch eine Probeaufnahme gezeigt, wie das Strahlenfeld im Verhältnis zur Bildplatte liegt, nachdem der Operateur zunächst den Strahlenkopf gegen die Bildkassette richtet, aber bevor die Anpassung des Strahlenfeldes, die mit der Vorrichtung nach der Erfindung möglich ist, durchgeführt worden ist. Die äußere Begrenzung des Strahlenfeldes wird mit den Linien 54 gezeigt.

Aus dem linken Teil der FIG 7 geht hervor, daß das Strahlenfeld in diesem Fall im Vergleich zur Bildplatte 52 teils falsch positioniert ist und teils eine Größeneinstellung fordert. Durch eine Parallelverschiebung des Strahlenfeldes sowie, wie oben beschrieben, eine Änderung der Größe durch Einstellen der Kollimatoren der Röntgenstrahlenquelle, wird eine im rechten Teil der FIG 7 gezeigte Lage, bei der die Form, Größe und Lage des Strahlenfeldes mit der Form, Größe und Lage der Bildplatte 52 ganz übereinstimmt, erreicht.

Der linke Teil der FIG 8 zeigt ein bei einer Probeaufnahme erhaltenes, trapezförmiges Bild 54 des Strahlenfeldes, wobei bei dieser Probeaufnahme der eine Teil der Bildkassette näher an dem Strahlenkopf mit der Röntgenstrahlenquelle als der andere Teil liegt, was für eine Korrektur eine Drehung des Strahlenkopfes erfordert. Nach einer Drehung des Strahlenkopfes, so wie es oben beschrieben worden ist, wird die im rechten Teil der FIG 8 gezeigte Lage erreicht, d.h., daß die Form, Größe und Lage des Strahlenfeldes mit der Form, Größe und Lage der Bildplatte 52 übereinstimmt.

In der FIG 9 werden auf der Bildplatte 52 die äußeren Konturen 61 eines Patienten gezeigt. Mit Hilfe der Erfindung kann danach die Erstreckung des Strahlenfeldes reduziert werden, so daß es den aktuellen Teil des Patienten genau abdeckt. Vergleiche hierzu FIG 9 rechts. Hierdurch wird die Strahlendosis auf die für die aktuelle Röntgenaufnahme unbedingt notwendige Dosis reduziert. Wie oben beschrieben, kann die Einstellung des Strahlenfeldes auf der Bildplatte in den in den FIG 7 bis 9 gezeigten Beispielen nach vorbestimmten Regeln, ohne daß der Operateur eingreifen muß, voll automatisch oder manuell erfolgen, indem auf einem Bildschirm ein Bild einer Bildplatte und eines Strahlenfeldes aufgenommen wird, so daß bei manuellen Einstellungen eine Rückkopplung erhalten wird, d.h., daß am Bildschirm die Effekte der Einstellungen beobachtbar sind. Die Einstellung des Strahlenfeldes kann auch halbautomatisch erfolgen, indem z.B. mit Hilfe einer sogenannten Maus oder durch die Verwendung eines sogenannten Berührungsschirmes die Lage der äußeren Begrenzungen 54 am Bildschirm markiert wird. Danach wird das Strahlenfeld gemäß entsprechenden Signalen mittels motorgetriebener Mittel zwecks Verschieben und Ausrichten des Strahlenkopfes und/oder Einstellen der Kollimatoren eingestellt.

Oben wird beschrieben, daß die Einstellung des Strahlenfeldes durch Justieren der Kollimatoren der Röntgenstrahlenquelle sowie durch Parallelverschieben und/oder Drehen des Strahlenkopfes erfolgt. Dabei wurde vorausgesetzt, daß der Abstand zwischen Strahlenkopf und Bildplatte konstant ist. Selbstverständlich kann auch dieser Abstand zwecks Veränderung des Strahlenfeldes auf der Bildplatte variiert werden.

Die Erfindung ist oben in Verbindung mit einem mobilen Röntgengerät beschrieben worden, die Erfindung ist selbstverständlich auch in Verbindung mit fest installierten Röntgenausrüstungen anwendbar. Insbesondere die in der in Verbindung mit der FIG 9 beschriebene Einstellung des Strahlenfeldes, bei der der interessante Teil des Patienten genau eingestellt worden ist, ist auch bei fest installierten Röntgenausrüstungen von großem Wert, da der Patient hier nicht unnötig hoher Strahlendosis ausgesetzt wird.

Auch Zahnröntgen ist für die Erfindung ein wichtiges Anwendungsgebiet, da hier eine mechanische Verbindung zwischen der Röntgenstrahlenquelle und der Bildplatte fehlt und man visuell nicht sehen kann, wie das Röntgenstrahlenbündel die Bildplatte treffen wird. Aus diesem Grund wird bei der heutigen Technik ein ausreichend großes Strahlenfeld eingestellt, damit die Bildplatte sicher getroffen wird.

## Patentansprüche

1. Röntgenaufnahmevorrichtung, umfassend einen Strahlenkopf (12, 24, 40) mit einer Röntgenröhre (1), die mit Kollimatoren (2, 44) zur Begrenzung eines ausgestrahlten Röntgenstrahlenbündels (17) versehen ist, bei dem der Strahlenkopf (12, 24, 40) zur Ausrichtung des Strahlen-bündels (17) im Raum translatorisch verschiebbar und/oder in Winkelrichtung drehbar ist, sowie eine digitale Bildplatte (4, 7, 30, 52), bestehend aus einer Mehrzahl von Pixeln (32), wobei eine mechanische Verbindung zwischen der Bildplatte (4, 7, 30, 52) und dem Strahlenkopf (12, 24, 40) fehlt, **gekennzeichnet durch** eine Abtasteinheit (36), die die Signale der Bildplatte (4, 7, 30, 52) abtastet, um zu bestimmen, welche Pixel (32) bei einer Probeaufnahme von dem Strahlenbündel (17) beleuchtet sind und in Abhängigkeit davon Steuersignale an Einstellmittel (42) und/oder an Verschieb- und/oder Drehmittel (38, 56, 58) abgibt, die abhängig von den Steuersignalen die Kollimatoren (2, 44) bzw. die Richtung des Strahlenkopfes (12, 24, 40) in Bezug auf die Bildplatte (4, 7, 30, 52) einstellen und/oder den Strahlenkopf (12, 24, 40) parallel zur Bildplatte (4, 7, 30, 52) zur optimalen Einstellung des Strahlenbündels (17) auf dieser bewegen.

2. Vorrichtungen nach Anspruch 1, **dadurch gekennzeichnet, daß** die Kollimatoren (44) motorgetrieben sind und daß die Einstellmittel (42) so angeordnet sind, daß sie automatisch die Motoren in Abhängigkeit einer abgetasteten Beleuchtung der Pixel (32) der Bildplatte (30) steuern.

3. Vorrichtung nach Anspruch 1, **dadurch gekennzeichnet, daß** die Drehmittel (38) eine motorgetriebene Antriebseinheit (48) zum automatischen Einstellen des Strahlenkopfes (40) in Abhängigkeit einer abgetasteten Beleuchtung der Bildplatte (30) steuern.

4. Vorrichtung nach einem der Ansprüche 1 bis 3, **dadurch gekennzeichnet, daß** die Einstellmittel (42) und die Verschieb- und Drehmittel (38) dazu vorgesehen sind, die Kollimatoren (2, 44) und/oder die Lage und Richtung des Strahlenkopfes (40) so einzustellen, daß die äußeren Begrenzungen (54) des Strahlenbündels (17) an der Bildplatte (4, 30, 52) im wesentlichen mit den äußeren Begrenzungen der Bildplatte (4, 30, 52) übereinstimmen.

5. Vorrichtung nach einem der Ansprüche 1 bis 3, **dadurch gekennzeichnet, daß** die Abtasteinheit (36) dazu vorgesehen ist, bei einer Probeaufnahme die erhaltenen äußeren Konturen (61) eines Patienten (6) zu bestimmen, sowie daß die Einstellmittel (42) und die Verschieb- und Drehmittel (38) dazu vorgesehen sind, das Strahlenfeld in Abhängigkeit von den Steuersignalen der Abtasteinheit auf der Bildplatte (4, 30, 52) an die äußeren Konturen (61) des Patienten (6) anzupassen.

## Claims

1. X-ray device, comprising an emitter head (12, 24, 40) with an X-ray tube (1) which is provided with collimators (2, 44) in order to restrict an emitted beam of X-rays (17), whereby the emitter head (12, 24, 40) can be displaced and/or rotated linearly in the space in the angular direction in order to align the beam of rays (17), and also a digital image plate (4, 7, 30, 52) consisting of a plurality of pixels (32), whereby there is no mechanical connection between the image plate (4, 7, 30, 52) and the emitter head (12, 24, 40),
**characterized by** a scanning unit (36) which scans the signals of the image plate (4, 7, 30, 52) in order to determine which pixels (32) are illuminated by the beam of rays (17) when an X-ray is being taken and, depending on this, issues control signals to setting facilities (42) and/or to displacement and/or rotating facilities (38, 56, 58) which, depending on the control signals, set the collimators (2, 44) or the direction of the emitter head (12, 24, 40) with reference to the image plate (4, 7, 30, 52) and/or move the emitter head (12, 24, 40) parallel to the image plate (4, 7, 30, 52) for optimum setting of the ray beam (17) on the latter.

2. Device according to Claim 1, **characterized in that**
the collimators (44) are motor-driven and that the setting facilities (42) are arranged such that they control the motors automatically as a function of a scanned illumination of the pixels (32) of the image plate (30).

3. Device according to Claim 1, **characterized in that**
the rotating facilities (38) control a motor-driven drive unit (48) for automatically setting the emitter head (40) as a function of a sampled illumination of the image plate (30).

4. Device according to one of Claims 1 to 3, **characterized in that**
the setting facilities (42) and the displacement and rotating facilities (38) are provided in order to set the collimators (2, 44) and/or the position and direction of the emitter head (40) such that the outer limits (54) of the ray beam (17) at the image plate (4, 30, 52) essentially coincide with the outer limits of the image plate (4, 30, 52).

5. Device according to one of Claims 1 to 3, **characterized in that**
the scanning unit (36) is provided in order to determine the outer contours obtained (61) for a patient (6) when an X-ray is being taken, and that the setting facilities (42) and the displacement and rotating facilities (38) are provided in order to adapt the ray beam to the outer contours (61) of the patient (6) on the image plate (4, 30, 52) as a function of the control signals of the scanning unit.

## Revendications

1. Appareil de radiographie comprenant une tête de rayonnement (12, 24, 40) avec un tube à rayons X (1), qui est équipé de collimateurs (2, 44) pour délimiter un faisceau de rayons X (17) émis, appareil dans lequel la tête de rayonnement (12, 24, 40) peut, en vue d'orienter le faisceau de rayons (17) dans l'espace, être déplacée par coulissement en translation et/ou être tournée dans une direction angulaire, l'ensemble comportant également une plaque d'image (4, 7, 30, 52) numérique composée d'un grand nombre de pixels (32), une liaison mécanique entre la plaque d'image (4, 7, 30, 52) et la tête de rayonnement (12, 24, 40) étant inexistante,
**caractérisé par** une unité d'exploration (36) qui explore les signaux de la plaque d'image (4, 7, 30, 52) en vue de déterminer quels pixels (32) sont exposés au faisceau de rayons (17) lors d'un cliché d'essai, et qui délivre en fonction de cela, des signaux de commande à des moyens de réglage (42) et/ou à des moyens de translation et/ou de rotation (38, 56, 58), qui, en fonction des signaux de commande, déplacent les collimateurs (2, 44) ou règlent l'orientation de la tête de rayonnement (12, 24, 40) par rapport à la plaque d'image (4, 7, 30, 52) et/ou déplacent la tête de rayonnement (12, 24, 40) parallèlement à la plaque d'image (4, 7, 30, 52) pour assurer un réglage optimal du faisceau de rayonnement (17) sur celle-ci.

2. Appareil selon la revendication 1, **caractérisé en ce que** les collimateurs (44) sont entraînés par moteur, et **en ce que** les moyens de réglage (42) sont disposés de manière à commander automatiquement les moteurs en fonction d'une exposition des pixels (32) de la plaque d'image (30), relevée par exploration.

3. Appareil selon la revendication 1, **caractérisé en ce que** les moyens de rotation (38) commandent une unité d'entraînement (48) à moteur, pour le réglage automatique de la tête de rayonnement (40) en fonction d'une exposition de la plaque d'image (30), relevée par exploration.

4. Appareil selon l'une des revendications 1 à 3, **caractérisé en ce que** les moyens de réglage (42) et les moyens de translation et de rotation (38) sont prévus pour régler les collimateurs (2, 44) et/ou la position et l'orientation de la tête de rayonnement (40) de façon telle, que les limites extérieures (54) du faisceau de rayons (17) au niveau de la plaque d'image (4, 30, 52) coilncident sensiblement avec les limites extérieures de la plaque d'image (4, 30, 52).

5. Appareil selon l'une des revendications 1 à 3, **caractérisé en ce que** l'unité d'exploration (36) est prévue pour déterminer les contours extérieurs (61) d'un patient (6) obtenus lors d'un cliché d'essai, et **en ce que** les moyens de réglage (42) et les moyens de translation et de rotation (38) sont prévus pour adapter le champ de rayonnement, sur la plaque d'image (4, 30, 52), aux contours extérieurs (61) du patient (6), en fonction des signaux de commande de l'unité d'exploration.
